(19) Europäisches Patentamt
European Patent Office
Office européen des brevets

(11) EP 4 591 886 A1

(12) EUROPEAN PATENT APPLICATION
published in accordance with Art. 153(4) EPC

(43) Date of publication:
**30.07.2025 Bulletin 2025/31**

(21) Application number: **23867461.8**

(22) Date of filing: **18.09.2023**

(51) International Patent Classification (IPC):
***A61K 47/68*** *(2017.01)* ***C07K 16/30*** *(2006.01)*
***A61K 39/395*** *(2006.01)* ***A61P 35/00*** *(2006.01)*

(52) Cooperative Patent Classification (CPC):
**A61K 39/395; A61K 47/68; A61P 35/00; C07K 16/30**

(86) International application number:
**PCT/CN2023/119471**

(87) International publication number:
**WO 2024/061173 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.09.2022 CN 202211138033**

(71) Applicants:
- **Shanghai Miracogen Inc**
  **Shanghai 201112 (CN)**
- **Lepu Biopharma Co., Ltd.**
  **Shanghai 201114 (CN)**

(72) Inventors:
- **JIN, Yongshuai**
  **Shanghai 201114 (CN)**
- **HU, Chaohong**
  **Shanghai 201112 (CN)**
- **SUI, Ziye**
  **Shanghai 201114 (CN)**

(74) Representative: **Ipsilon**
**11 rue Saint-Georges**
**75009 Paris (FR)**

Remarks:
The complete document including Reference Table(s) and the Sequence Listing(s) can be downloaded from the EPO website

# (54) TREATMENT OF NASOPHARYNGEAL CARCINOMA BY USING ANTIBODY CONJUGATE TARGETING EGFR

(57) The present invention relates to a treatment of nasopharyngeal carcinoma by using an antibody conjugate targeting EGFR. Specifically, it is found that antibody-drug conjugate MYK-3 administered at 2.3 mg/kg once every three weeks can effectively treat advanced EGFR-positive and HER2-negative gastric cancer, recurrent metastatic nasopharyngeal carcinoma, recurrent or metastatic head and neck squamous cell carcinoma, advanced EGFR-positive biliary tract adenocarcinoma, advanced EGFR-positive non-small cell lung cancer, and other cancers. Compared with a lower dose of 2.0 mg/kg, 2.3 mg/kg of MYK-3 in clinical use demonstrated a good tumor inhibitory or delaying effect in patients and controllable adverse effects, suggesting good safety.

EP 4 591 886 A1

## Description

### TECHNICAL FIELD

The present disclosure relates to the field of pharmaceutical chemistry, and in particular to the use of an antibody-drug conjugate, more specifically to the treatment of nasopharyngeal carcinoma with an antibody conjugate targeting EGFR.

### BACKGROUND ART

Epidermal Growth Factor Receptor (EGFR, also known as HER1 and c-ErbB1) is a cell surface receptor of the epidermal growth factor family, and is a transmembrane glycoprotein with a molecular weight of 170 kD composed of 1186 amino acid residues. EGFR belongs to the type I tyrosine kinase receptor ErbB subfamily (ErbBs 1-4) and has tyrosine kinase activity. EGFR is stably expressed in many epithelial tissues, including skin and hair follicles. Abnormal expression of epidermal growth factor receptor or its activation due to mutation may lead to canceration. Many solid tumors have been found to overexpress epidermal growth factor receptor, such as colorectal cancer, head and neck cancer, lung cancer, ovarian cancer, cervical cancer, bladder cancer, and esophageal cancer. Growth factors such as transforming growth factor $\alpha$ and epidermal growth factor are endogenous ligands of epidermal growth factor receptor. These ligands bind to epidermal growth factor receptor, activate the intracellular tyrosine protein kinase activity of the receptor, and initiate a number of downstream signal transduction pathways, thus regulating the growth and differentiation of normal cells, increasing the invasion of tumor cells, promoting angiogenesis and inhibiting the apoptosis of tumor cells. These characteristics that epidermal growth factor receptor is overexpressed in tumors and plays an important role in the growth and differentiation of tumor cells make epidermal growth factor receptor a promising target for tumor treatment.

Antibody-drug conjugates (ADCs) are a new class of biological drugs formed by conjugating a monoclonal antibody (mAb) targeting a specific antigen to a small molecule drug (payload) with potent cytotoxicity via a linker. An ADC drug specifically identifies a target of a cancer cell and guides a small molecule drug to reach the target of the cancer cell via an antibody, then enters the cancer cell by endocytosis, and releases a cytotoxic drug to specifically kill the cancer cell. Since being put forward, ADCs have been undergoing constant innovation and optimization and have achieved success in tumor treatment.

At present, anti-EGFR antibody-drug conjugates have been continuously developed, for example, the anti-EGFR antibody-drug conjugate MYK-3 in the granted patent CN 106999606 B. However, the clinical dosage of the anti-EGFR antibody-drug conjugate MYK-3 has not been deeply studied and is still need to explored and solved.

### SUMMARY OF THE INVENTION

By exploration via extensive experiments and creative efforts, the inventors of the present application have found that the antibody-drug conjugate MYK-3 administered at 2.3 mg/kg once every three weeks in clinical use can effectively treat advanced EGFR-positive and HER2-negative gastric cancer, recurrent metastatic nasopharyngeal carcinoma, recurrent or metastatic head and neck squamous cell carcinoma, advanced EGFR-positive biliary tract adenocarcinoma, advanced EGFR-positive non-small cell lung cancer, and other cancers. The inventors have found that compared with a lower dose of 2.0 mg/kg, a dose of 2.3 mg/kg of MYK-3 in clinical use demonstrated a good tumor inhibitory or delaying effect in patients and controllable adverse effects, suggesting good safety.

To this end, in one aspect of the present disclosure, the present disclosure provides an antibody-drug conjugate, or a pharmaceutically acceptable salt or solvate thereof, or a solvate of the salt, for use in treating an epidermal growth factor receptor (EGFR)-associated disease, wherein the antibody-drug conjugate has a structure represented by formula I:

$$Ab\text{-}(L\text{-}D)_p \qquad \text{formula I}$$

in which:

Ab represents an anti-epidermal growth factor receptor antibody, and the anti-epidermal growth factor receptor antibody comprises a heavy chain and a light chain, wherein CDR1, CDR2, and CDR3 in a heavy chain variable region comprise the sequences as set forth in SEQ ID NO: 5, SEQ ID NO: 6, and SEQ ID NO: 7, respectively, or mutants thereof, and CDR1, CDR2, and CDR3 in a light chain variable region comprise the sequences as set forth in SEQ ID NO: 12, SEQ ID NO: 13, and SEQ ID NO: 14, respectively, or mutants thereof;

L represents a linker, and the linker is 6-maleimidocaproyl-valine-citrulline-p-aminobenzyloxycarbonyl (MC-vc-PAB);

D represents a cytotoxic agent, and the cytotoxic agent is MMAE; and

p is any numerical value selected from 1-8 (e.g., 1, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, or 8, or e.g., 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, or 7.5-8),

wherein the total dosage of the antibody-drug conjugate, or the pharmaceutically acceptable salt or solvate thereof, or the solvate of the salt every three weeks is 2.1-2.4 mg/kg.

In another aspect of the present disclosure, the present disclosure further provides a method for treating an epidermal growth factor receptor (EGFR)-associated disease, comprising administering an antibody-drug conjugate, or a pharmaceutically acceptable salt or solvate thereof, or a solvate of the salt to a subject, wherein
the antibody-drug conjugate has a structure represented by formula I:

$$\text{Ab-(L-D)}_p \qquad \text{formula I}$$

in which:

Ab represents an anti-epidermal growth factor receptor antibody, and the anti-epidermal growth factor receptor antibody comprises a heavy chain and a light chain, wherein CDR1, CDR2, and CDR3 in a heavy chain variable region comprise the sequences as set forth in SEQ ID NO: 5, SEQ ID NO: 6, and SEQ ID NO: 7, respectively, or mutants thereof, and CDR1, CDR2, and CDR3 in a light chain variable region comprise the sequences as set forth in SEQ ID NO: 12, SEQ ID NO: 13, and SEQ ID NO: 14, respectively, or mutants thereof;

L represents a linker, and the linker is 6-maleimidocaproyl-valine-citrulline-p-aminobenzyloxycarbonyl (MC-vc-PAB);

D represents a cytotoxic agent, and the cytotoxic agent is MMAE; and

p is any numerical value selected from 1-8 (e.g., 1, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, or 8, or e.g., 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, or 7.5-8),

wherein the total dosage of the antibody-drug conjugate, or the pharmaceutically acceptable salt or solvate thereof, or the solvate of the salt every three weeks is 2.1-2.4 mg/kg.

In formula I, L-D means that the linker is linked to the cytotoxic agent via a covalent bond to form an L-D molecule; and Ab-$(L-D)_p$ means that p L-D molecules are conjugated to Ab via covalent bonds.

In the present disclosure, the drug-to-antibody ratio (DAR) refers to the number of drug (or known as cytotoxic agent) molecules conjugated to the antibody (e.g., p in formula I). The number of drug molecules contained in the antibody-drug conjugate described herein can be either an integer or a decimal. The number, whether an integer or a decimal, refers to the average number of drug molecules conjugated to each antibody. "p is any numerical value between 1 and 8" means that p may be either any integer between 1 and 8 (including endpoints 1 and 8) or any decimal between 1 and 8, e.g., 4.1. Moreover, those skilled in the art can understand that even if the same preparation method is used, the DAR values of antibody-drug conjugates prepared in different batches are not necessarily the same, for example, they can fluctuate within a range of no more than 0.5 up and down.

The drug-to-antibody ratio (DAR) can be determined by conventional means, such as mass spectrometry, ELISA assay, HIC, and HPLC. ADCs may also be measured for quantitative distribution in terms of p. In some cases, the separation of homogeneous ADCs with p as a certain numerical value from ADCs with other drug loads, followed by purification and verification can be achieved by means such as HIC, reversed-phase HPLC or electrophoresis.

The structural formula of MC-vc-PAB is as shown below:

MC-vc-PAB

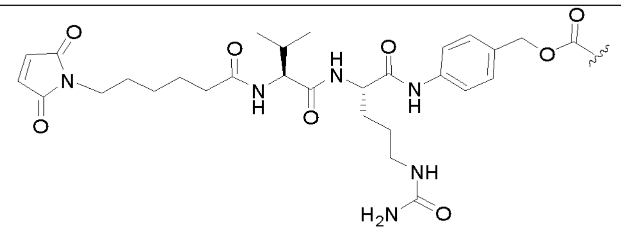

The structural formula of MMAE is as shown below:

MMAE

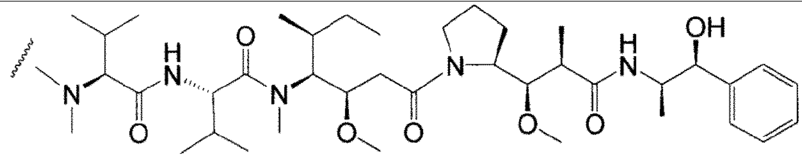

In formula I, L-D is MC-vc-PAB-MMAE, the structural formula of which is as shown below:

MC-vc-PAB-MMAE

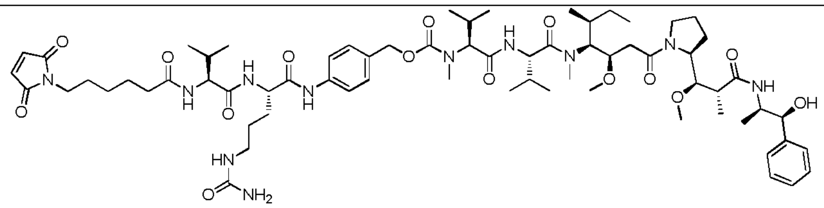

When the above L-D, i.e., MC-vc-PAB-MMAE, is conjugated to Ab via a covalent bond, ADC is formed by conjugating succinimide at one end of L-D to thiol in the antibody, and the structural formula of the formed ADC is as shown below:

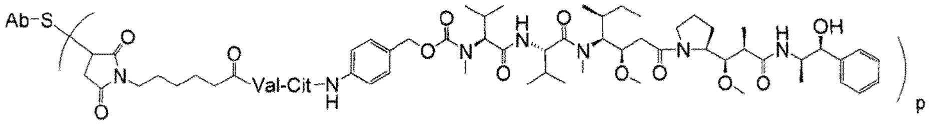

In the formed ADC above, the antibody Ab is linked to a carbon atom of the succinimide at the end of L-D via -S-, and the -S- is not a thiol group additionally introduced into Ab, but a thiol group contained in the antibody itself after the antibody Ab is reduced to break a disulfide bond.

In some embodiments, the total dosage of the antibody-drug conjugate, or the pharmaceutically acceptable salt or solvate thereof, or the solvate of the salt every three weeks is 2.2-2.4 mg/kg.

In some embodiments, the total dosage of the antibody-drug conjugate, or the pharmaceutically acceptable salt or solvate thereof, or the solvate of the salt every three weeks is 2.3 mg/kg.

It should be noted that "every three weeks" means that 3 weeks is one treatment cycle, and "total dosage every three weeks" means the total dosage in this treatment cycle (i.e., every three weeks). For example, if the total dosage every three weeks is 2.3 mg/kg, the dosage regimen may be a low frequency of administration with a high single dose (for example, the single dose is 2.3 mg/kg, and the frequency of administration is once every three weeks), or may also be a high frequency of administration with a low single dose (for example, the single dose is 0.77 mg/kg, and the frequency of administration is once every 7 days, that is, once every week). The specific number of treatment cycles may be, for example, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, or at least 40, or even more. In some embodiments, the specific configuration or selection of the number of treatment cycles is based on the principle of continuous treatment until the disease progresses. In addition, it can be understood by those skilled in the art that the dosage can slightly fluctuate within a range of no more than 0.05 mg/kg above and below the dose.

In some embodiments, the single dose of the antibody-drug conjugate, or the pharmaceutically acceptable salt or solvate thereof, or the solvate of the salt is 2.1-2.4 mg/kg (preferably 2.2-2.4 mg/kg, more preferably 2.3 mg/kg), and the

frequency of administration is once every three weeks. In some embodiments, the dosage is given on the 1st day of the every three weeks.

In some embodiments, the single dose of the antibody-drug conjugate, or the pharmaceutically acceptable salt or solvate thereof, or the solvate of the salt is 0.7-1.4 mg/kg (e.g., 0.7-1.1 mg/kg, specifically e.g., 0.77 mg/kg or 1.1 mg/kg), and the frequency of administration is once every 7-10 days.

In some embodiments, the mode of administration of the antibody-drug conjugate, or the pharmaceutically acceptable salt or solvate thereof, or the solvate of the salt is intravenous drip (or known as intravenous administration, intravenous injection, etc.).

In some embodiments, the subject to be administered with the antibody-drug conjugate, or the pharmaceutically acceptable salt or solvate thereof, or the solvate of the salt is a human.

In some embodiments, the subject to be administered with the antibody-drug conjugate, or the pharmaceutically acceptable salt or solvate thereof, or the solvate of the salt is a patient suffering from an epidermal growth factor receptor (EGFR)-associated disease.

In some embodiments, the subject to be administered with the antibody-drug conjugate, or the pharmaceutically acceptable salt or solvate thereof, or the solvate of the salt is a patient who had been previously treated with an anti-EGFR drug, and preferably, the anti-EGFR drug is an anti-EGFR antibody.

In some embodiments, the subject to be administered with the antibody-drug conjugate, or the pharmaceutically acceptable salt or solvate thereof, or the solvate of the salt is a patient who overexpresses EGFR.

In some embodiments, the subject to be administered with the antibody-drug conjugate, or the pharmaceutically acceptable salt or solvate thereof, or the solvate of the salt is a patient with recurrent metastatic nasopharyngeal carcinoma (NPC) who had previously received at least a first-line platinum-containing regimen and systematic treatment with a PD-1/PD-L1 inhibitor, during which progression occurred or after which recurrence or intolerance occurred.

In some embodiments, the subject to be administered with the antibody-drug conjugate, or the pharmaceutically acceptable salt or solvate thereof, or the solvate of the salt is a patient with recurrent metastatic nasopharyngeal carcinoma (NPC) who had previously received at least a second-line systemic chemotherapy and a PD-1/PD-L1 treatment and failed. In some embodiments, the chemotherapy regimen of the previously received at least second-line systemic chemotherapy should include at least a platinum-containing regimen, gemcitabine, and taxane drugs/capecitabine.

In some embodiments, the subject is a human.

In some embodiments, the subject is a patient suffering from an epidermal growth factor receptor (EGFR)-associated disease.

In some embodiments, the subject is a patient who had been previously treated with an anti-EGFR drug, and preferably, the anti-EGFR drug is an anti-EGFR antibody.

In some embodiments, the subject is a patient who overexpresses EGFR.

In some embodiments, the subject is a patient with recurrent metastatic nasopharyngeal carcinoma (NPC) who had previously received at least a first-line platinum-containing regimen and systematic treatment with a PD-1/PD-L1 inhibitor, during which progression occurred or after which recurrence or intolerance occurred.

In some embodiments, the subject is a patient with recurrent metastatic nasopharyngeal carcinoma (NPC) who had previously received at least a second-line systemic chemotherapy and a PD-1/PD-L1 treatment and failed. In some embodiments, the chemotherapy regimen of the previously received at least second-line systemic chemotherapy should include at least a platinum-containing regimen, gemcitabine, and taxane drugs/capecitabine.

In some embodiments, the taxane drugs include paclitaxel, docetaxel, albumin-bound paclitaxel, and paclitaxel liposome.

In some embodiments, the platinum-containing regimen includes cisplatin, carboplatin, nedaplatin, etc.

In some embodiments, the platinum-containing regimen includes, but is not limited to, the following treatment regimens:

cisplatin + gemcitabine (preferred); or

platinum (such as cisplatin or carboplatin) + taxane (such as docetaxel or paclitaxel); or

platinum + anti-EGFR monoclonal antibody (such as cetuximab or nimotuzumab).

In some embodiments, the epidermal growth factor receptor (EGFR)-associated disease is a cancer.

In some embodiments, the cancer is selected from nasopharyngeal carcinoma, head and neck squamous cell carcinoma, biliary tract cancer, esophageal cancer, duodenal cancer, colorectal cancer, colon cancer, rectal cancer, head and neck cancer, lung cancer (such as non-small cell lung cancer), ovarian cancer, cervical cancer, bladder cancer, esophagus cancer, breast cancer, renal cancer, prostate cancer, gastric cancer, pancreatic cancer, and glioma.

In some embodiments, the cancer is selected from nasopharyngeal carcinoma, head and neck squamous cell

carcinoma, non-small cell lung cancer, biliary tract cancer, gastric cancer, esophageal cancer, and duodenal cancer.

In some embodiments, the cancer is nasopharyngeal carcinoma.

In some embodiments, the cancer is a heavily pretreated advanced solid tumor.

In some embodiments, the cancer is an advanced EGFR-positive solid tumor that recurs or metastasizes after multiple previous tumor-associated treatments.

In some embodiments, the cancer is selected from advanced EGFR-positive and HER2-negative gastric cancer, recurrent metastatic nasopharyngeal carcinoma, recurrent or metastatic head and neck squamous cell carcinoma, advanced EGFR-positive biliary tract adenocarcinoma, advanced EGFR-positive non-small cell lung cancer, etc. "EGFR-positive" means that EGFR is overexpressed, and "HER2-negative" means that HER2 is not overexpressed.

In some embodiments, the cancer is recurrent metastatic nasopharyngeal carcinoma.

In some embodiments, p is any numerical value selected from 2-6.

In some embodiments, p is any numerical value selected from 3-5.

In some embodiments, FR1, FR2, FR3, and FR4 regions in a heavy chain variable region of the anti-epidermal growth factor receptor antibody comprise the sequences as set forth in SEQ ID NO: 8, SEQ ID NO: 9, SEQ ID NO: 10, and SEQ ID NO: 11, respectively, or mutants thereof.

In some embodiments, FR1, FR2, FR3, and FR4 regions in a light chain variable region of the anti-epidermal growth factor receptor antibody comprise the sequences as set forth in SEQ ID NO: 15, SEQ ID NO: 16, SEQ ID NO: 17, and SEQ ID NO: 18, respectively, or mutants thereof.

In some embodiments, a heavy chain constant region of the anti-epidermal growth factor receptor antibody is selected from human IgG, IgM, IgA, IgD, and IgE constant regions, or mutants of the constant regions.

In some embodiments, the IgG is selected from IgG1, IgG2, IgG3, and IgG4.

In some embodiments, a light chain constant region of the anti-epidermal growth factor receptor antibody is human lambda constant region or kappa constant region, or a mutant of the constant region.

In some embodiments, the sequence of the heavy chain variable region of the anti-epidermal growth factor receptor antibody comprises the sequence as set forth in SEQ ID NO: 1, or a sequence with more than 70%, preferably more than 75%, 80%, 85%, 90%, 95%, or 99% homology with the sequence as set forth in SEQ ID NO: 1.

In some embodiments, the sequence of the heavy chain variable region of the anti-epidermal growth factor receptor antibody is as set forth in SEQ ID NO: 1.

In some embodiments, the sequence of the light chain variable region of the anti-epidermal growth factor receptor antibody comprises the sequence as set forth in SEQ ID NO: 2, or a sequence with more than 70%, preferably more than 75%, 80%, 85%, 90%, 95%, or 99% homology with the sequence as set forth in SEQ ID NO: 2.

In some embodiments, the sequence of the light chain variable region of the anti-epidermal growth factor receptor antibody is as set forth in SEQ ID NO: 2.

In some embodiments, the sequence of the heavy chain constant region of the anti-epidermal growth factor receptor antibody comprises the sequence as set forth in SEQ ID NO: 3, or a sequence with more than 70%, preferably more than 75%, 80%, 85%, 90%, 95%, or 99% homology with the sequence as set forth in SEQ ID NO: 3.

In some embodiments, the sequence of the heavy chain constant region of the anti-epidermal growth factor receptor antibody is as set forth in SEQ ID NO: 3.

In some embodiments, the sequence of the light chain constant region of the anti-epidermal growth factor receptor antibody comprises the sequence as set forth in SEQ ID NO: 4, or a sequence with more than 70%, preferably more than 75%, 80%, 85%, 90%, 95%, or 99% homology with the sequence as set forth in SEQ ID NO: 4.

In some embodiments, the sequence of the light chain constant region of the anti-epidermal growth factor receptor antibody is as set forth in SEQ ID NO: 4.

Beneficial Effects

The object of the present disclosure is to determine the clinical dosage and indications of MYK-3. Through the phase I/II clinical study, the inventors have found that MYK-3 administered at 2.3 mg/kg once every three weeks can treat advanced EGFR-positive and HER2-negative gastric cancer, recurrent metastatic nasopharyngeal carcinoma, recurrent or metastatic head and neck squamous cell carcinoma, advanced EGFR-positive biliary tract adenocarcinoma, and advanced EGFR-positive non-small cell lung cancer. In the expanded sequence for recurrent metastatic nasopharyngeal carcinoma, MYK-3 exhibited good therapeutic effect. The inventors have found that by comparing with the doses of 2.0 mg/kg and 2.5 mg/kg, 2.3 mg/kg of MYK-3 in clinical use demonstrated a good tumor inhibitory or delaying effect in patients and controllable adverse effects, suggesting good safety.

## BRIEF DESCRIPTION OF THE DRAWINGS

Figure 1: an HIC-HPLC chromatogram of the determination of the drug/antibody ratio of an antibody-drug conjugate.
Figure 2: the determination of a monoclonal antibody and an antibody-drug conjugate for *in vitro* cell activity, wherein ◦ represents the monoclonal antibody BA03 and ▲ represents the antibody-drug conjugate MYK-3.
Figure 3: the inhibitory activity of MYK-3 on the growth of the colon cancer cells HT-29, wherein ◦ represents the monoclonal antibody BA03 and ▲ represents the antibody-drug conjugate MYK-3.
Figure 4: the inhibitory activity of MYK-3 on the growth of the glioma cancer cells U87-MG, wherein ◦ represents the monoclonal antibody BA03 and ▲ represents the antibody-drug conjugate MYK-3.
Figure 5: the inhibitory activity of MYK-3 on the growth of the lung cancer cells A549, wherein o represents the monoclonal antibody BA03 and ▲ represents the antibody-drug conjugate MYK-3.
Figure 6: the inhibitory activity of MYK-3 on the growth of the KRAS-mutant colon cancer cells LoVo, wherein ◊ represents the monoclonal antibody Erbitux and ▲ represents the antibody-drug conjugate MYK-3.

## DETAILED DESCRIPTION OF EMBODIMENTS

Embodiments of the present disclosure will be described in detail below with reference to examples; however, those skilled in the art will understand that the following examples are only used to illustrate the present disclosure and should not be regarded as limiting the scope of the present disclosure. For examples in which specific conditions are not specified, they are carried out according to the conventional conditions or the conditions recommended by the manufacturer. The reagents or instruments used without indicating the manufacturer are all commercially available conventional products.

In the present disclosure, the scientific and technical terms used herein have the meanings that are commonly understood by those skilled in the art unless otherwise specified. In addition, the terms and laboratory operation steps related to the protein and nucleic acid chemistry, molecular biology, cell and tissue culture, microbiology and immunology used herein are all terms and conventional steps that are widely used in the corresponding art. Moreover, for better understanding of the present disclosure, definitions and explanations of related terms are provided below.

In the present disclosure, any numerical range should be interpreted as including any value within the range or any subrange unless otherwise specified.

In the present disclosure, the term "antibody" refers to an immunoglobulin molecule that generally consists of two pairs of identical polypeptide chains (each pair has one "light" (L) chain and one "heavy" (H) chain). Antibody light chains can be divided into two categories, i.e., $\kappa$ and $\lambda$. Heavy chains can be divided into five types, i.e., $\mu$, $\delta$, $\gamma$, $\alpha$, or $\varepsilon$. According to different heavy chains, antibodies can be divided into five categories, i.e., IgM, IgD, IgG, IgA, and IgE. Within light and heavy chains, the variable and constant regions are joined by a "J" region of about 12 or more amino acids, with the heavy chain further comprising a "D" region of about 3 or more amino acids. Each heavy chain consists of a heavy chain variable region ($V_H$) and a heavy chain constant region ($C_H$). The heavy chain constant region consists of three domains ($C_H1$, $C_H2$, and $C_H3$). Each light chain consists of a light chain variable region ($V_L$) and a light chain constant region ($C_L$). The light chain constant region consists of one domain, i.e., $C_L$. The constant regions of the antibodies can mediate the binding of the immunoglobulin to host tissues or factors, including various cells of the immune system (e.g., effector cells) and component C1q of the complement system. The $V_H$ and $V_L$ regions can be further subdivided into regions of hypervariability, termed complementarity determining regions (CDRs), interspersed with regions that are more conserved, termed framework regions (FRs). Each $V_H$ and $V_L$ consists of three CDRs and four FRs, arranged from amino-terminus to carboxy-terminus in the following order: FR1, CDR1, FR2, CDR2, FR3, CDR3, FR4. The variable regions ($V_H$ and $V_L$) of each heavy/light chain pair form antibody binding sites, respectively. Distribution of amino acids in various regions or domains follows the definitions in: Kabat Sequences of Proteins of Immunological Interest (National Institutes of Health, Bethesda, Md. (1987 and 1991)), or Chothia & Lesk (1987) J. Mol. Biol. 196:901-917; and Chothia et al. (1989) Nature 342:878-883.

The antibody of the present disclosure can be prepared by various methods known in the art, for example, obtained by genetic engineering recombinant technology. For example, DNA molecules encoding the heavy chain and light chain genes of the antibody of the present disclosure are obtained by chemical synthesis or PCR amplification. The obtained DNA molecules are inserted into an expression vector, which is then transfected into host cells. Then, the transfected host cells are cultured under specific conditions, and the antibody of the present disclosure is expressed.

In the present disclosure, algorithms for determining the percentage of sequence homology and sequence similarity are, for example, BLAST and BLAST 2.0 algorithms, which are described in Altschul et al. (1977) Nucl. Acid. Res. 25:3389-3402 and Altschul et al. (1990) J. Mol. Biol. 215:403-410, respectively. BLAST and BLAST 2.0 can be used to determine the percentage of amino acid sequence homology of the present disclosure, using parameters such as those described in the literatures or default parameters. Softwares for performing BLAST analyses are publicly available through the National Center for Biotechnology Information (NCBI).

In the present disclosure, the amino acid sequence having at least 70% sequence homology with an amino acid sequence includes a polypeptide sequence essentially identical to the amino acid sequence, e.g., those sequences

comprising at least 70% sequence homology, preferably at least 75%, 80%, 85%, 86%, 87%, 88%, 89%, 90%, 91%, 92%, 93%, 94%, 95%, 96%, 97%, 98%, 99%, or more sequence homology with the polypeptide sequence of the present disclosure when using the method described herein (e.g., BLAST analyses using standard parameters).

In the present disclosure, a mutant of the amino acid sequence refers to a sequence having more than 70%, for example, more than 75%, 80%, 85%, 90%, 91%, 92%, 93%, 94%, 95%, 96%, 97%, 98%, or 99% homology with the amino acid sequence, e.g., a sequence with three, two, or one substituted, deleted or added amino acids. Preferably, no more than three amino acids are substituted, added or deleted. More preferably, no more than two amino acids are substituted, added or deleted. Most preferably, no more than one amino acid is substituted, added or deleted.

A "substitution" variant is a variant in which at least one amino acid residue in the natural sequence is removed and a different amino acid is inserted in the same position therein. The substitution may be single, wherein only one amino acid in the molecule is substituted; or may be multiple, wherein two or more amino acids in the same molecule are substituted. The multiple substitutions may be at consecutive sites. Similarly, an amino acid can be substituted by multiple residues, wherein such variants comprise both substitutions and insertions. An "insertion" (or "addition") variant is a variant in which one or more amino acids are inserted immediately adjacent to amino acids at a specific position in a natural sequence. Immediately adjacent to an amino acid means linked to either the $\alpha$-carboxyl or $\alpha$-amino functional group of the amino acid. A "deletion" variant is a variant in which one or more amino acids in the natural amino acid sequence are removed. Generally, a deletion variant has one or two amino acids deleted in a specific region of its molecule.

In the present disclosure, overexpression of EGFR means that the expression level of EGFR is increased as compared with the expression level of EGFR on the surface of normal epithelial cells. Specifically, cells can be further divided into those with a high expression, a moderate expression, and a low expression. For example, DiFi cells belong to a cell line with high EGFR expression, LoVo cells belong to a cell line with moderate EGFR expression, and HT-29 cells belong to a cell line with low EGFR expression (Wild, R., et al., Mol. Cancer Rher 2006:5(1), p104-113, Cetuximab preclinical antitumor activity (monotherapy and combination based) is not predicted by relative total or activated epidermal growth factor receptor tumor expression levels).

In the present disclosure, the KRAS gene has the same meaning as the K-RAS gene, which is a member of the RAS gene family and encodes the K-ras protein, and is related to the generation, proliferation, migration, diffusion and angiogenesis of various tumors. Common mutation sites therein are codon 12 and codon 13 of exon 2 and codon 61 of exon 3 in the K-RAS gene, wherein there are 7 mutation hotspots: G12C, G12R, G12S, G12V, G12D, G12A, and G13V/D, and these seven mutations account for 90% or more. In one embodiment of the present disclosure, the tumor is a tumor with KRAS gene mutation related to EGFR overexpression.

In the present disclosure, 20 conventional amino acids and abbreviations thereof follow conventional usages. See Immunology-A Synthesis (2nd edition, E.S. Golub and D.R. Gren, Eds., Sinauer Associates, Sunderland, Mass. (1991)), which is incorporated herein by reference.

In the present disclosure, fewer than the theoretical maximum number of drug modules are conjugated to the antibody in the conjugation reaction. Generally, the antibody does not contain many free and reactive cysteine thiol groups, which can link drug modules; and in fact, most cysteine thiol groups in the antibody exist as disulfide bridges. In certain embodiments, the antibody can be reduced with a reducing agent such as dithiothreitol (DTT) or tris(2-carboxyethyl) phosphine (TCEP) under partial or complete reducing conditions to produce reactive cysteine thiol groups.

In the present disclosure, the term "pharmaceutically acceptable salt" refers to (i) salts formed from acidic functional groups present in the conjugate provided by the present disclosure and appropriate inorganic or organic cations (bases), including but not limited to alkali metal salts, such as sodium salts, potassium salts, and lithium salts; alkaline earth metal salts, such as calcium salts and magnesium salts; other metal salts, such as aluminum salts, iron salts, zinc salts, copper salts, nickel salts, and cobalt salts; inorganic base salts, such as ammonium salts; and organic base salts, such as tert-octylamine salts, dibenzylamine salts, morpholine salts, glucosamine salts, alkyl phenylglycinate salts, ethylenediamine salts, N-methylglucosamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenylethylamine salts, piperazine salts, tetramethylamine salts, and tris(hydroxymethyl)aminomethane salts; and (ii) salts formed from basic functional groups present in the conjugate provided by the present disclosure and appropriate inorganic or organic anions (acids), including but not limited to hydrohalides, such as hydrofluorides, hydrochlorides, hydrobromides, and hydroiodides; inorganic acid salts, such as nitrates, perchlorates, sulfates, and phosphates; lower alkane sulfonates, such as methanesulfonates, trifluoromethanesulfonates, and ethanesulfonates; arylsulfonates, such as benzenesulfonates and p-benzenesulfonates; organic acid salts, such as acetates, malates, fumarates, succinates, citrates, tartrates, oxalates, and maleates; and amino acid salts, such as glycine salts, trimethylglycine salts, arginine salts, ornithine salts, glutamic acid salts, and aspartic acid salts.

Pharmaceutically acceptable salts can be obtained using standard procedures well known in the art, for example, by reacting a sufficient amount of a basic substance with a suitable acid providing a pharmaceutically acceptable anion, or by reacting a sufficient amount of an acidic substance with a suitable base providing a pharmaceutically acceptable cation.

In the present disclosure, a solvate refers to the following form of the antibody-drug conjugate of the present

disclosure: a solid or liquid complex formed by coordination of the antibody-drug conjugate with solvent molecules. A hydrate is a specific form of solvate, which has coordinated water molecules. In the present disclosure, the hydrate is a preferred solvate.

In the present disclosure, the term "treatment" generally refers to obtaining a desired pharmacological and/or physiological effect. The effect may be prophylactic on the basis of the complete or partial prevention of a disease or symptoms thereof; and/or the effect may be therapeutic on the basis of the partial or complete stabilization or cure of the disease and/or side effects due to the disease. As used herein, "treatment" covers any treatment of a disease in a patient, including: (a) prevention of a disease or symptom in a patient who is susceptible to the disease or symptom but has not been diagnosed with the disease; (b) suppressing a symptom of a disease, i.e., preventing the development thereof; or (c) relieving a symptom of a disease, i.e., causing the disease or symptom to resolve.

In the present disclosure, the term "subject" refers to a vertebrate. In certain embodiments, a vertebrate refers to a mammal. Mammals include, but are not limited to, livestock (such as cattle), pets (such as cats, dogs, and horses), primates, mice, and rats. In certain embodiments, a mammal refers to a human.

The antibody BA03 in the present disclosure is BA03 in China invention patent application CN 103772504 A. For the preparation method therefor, reference can be made to Example 3 in the patent application. The sequence of each part of the antibody is as follows.

The heavy chain variable region sequence is:

QVQLQESGPGLVKPSETLSLTCTVSGFSLSNYDVHWVRQAPGKGLEWLGVIWSGGNTDYNTPFTSRLTISVDTSKNQFSLKLSSVTAADTAVYYCARALDYYDYEFAYWGQGTLVTVSS (SEQ ID NO: 1),

wherein the underlined parts are CDR1 (SEQ ID NO: 5), CDR2 (SEQ ID NO: 6), and CDR3 (SEQ ID NO: 7), respectively; and

the non-underlined parts are FR1 (SEQ ID NO: 8), FR2 (SEQ ID NO: 9), FR3 (SEQ ID NO: 10), and FR4 (SEQ ID NO: 11), respectively.

The light chain variable region sequence is:

EIVLTQSPDFQSVTPKEKVTITCRASQSIGTNIHWYQQKPDQSPKLLIKYASESISGIPSRFSGSGSGTDFTLTINSLEAEDAATYYCQQNNEWPTSFGQGTKLEIK (SEQ ID NO: 2),

wherein the underlined parts are CDR1 (SEQ ID NO: 12), CDR2 (SEQ ID NO: 13), and CDR3 (SEQ ID NO: 14), respectively; and
the non-underlined parts are FR1 (SEQ ID NO: 15), FR2 (SEQ ID NO: 16), FR3 (SEQ ID NO: 17), and FR4 (SEQ ID NO: 18), respectively.

The heavy chain constant region sequence is:

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 3).

The light chain constant region sequence is:

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 4).

The present disclosure will be further explained with reference to specific examples below, and these examples do not limit the scope of the present disclosure.

**Example 1:** Method for preparing antibody-drug conjugate

10 mg of the antibody BA03 was taken and exchanged into a reducing buffer (25 mM sodium borate, pH 8.0, 25 mM NaCl, 5 mM EDTA) by using a 15 mL 30 KD ultrafiltration device for a total of three instances of exchange. The final volume was about 1 mL, which was transferred to a new Eppendorf centrifuge tube (weighed) and weighed. The protein concentration was detected, and the total protein amount was calculated. 2.5 folds by number of moles of DTT was added to the antibody, and the mixture was maintained at room temperature for 2 hours with continuous mixing. A total of three instances of exchange into a conjugate buffer (50 mM Tris, pH 7.2, 150 mM NaCl, 5 mM EDTA) were carried out by using a 15 mL 30 KD ultrafiltration device. The concentrated solution was taken, and the protein concentration was measured according to A280, followed by weighing, and total protein amount calculation. 10 μL of a sample was taken, and the number of free thiol groups was determined by Ellman's method.

The molar concentration of free thiol groups thereof was calculated by the following formula:

$$C_{thiol} = \frac{A412 \times 112}{b \times 14150} \ (M)$$

b: the optical path length of a cuvette (usually 1 cm).

According to the molar concentration of free thiol groups and the total volume of the protein solution, the number of moles of free thiol groups was calculated.

vc-MMAE (purchased from Shanghai Haoyuan Chemexpress Co., Ltd., article number HY-15575) (dissolved in DMSO) was added to the reduced antibody in an amount of 1.1 folds of the number of moles of free thiol groups, and the mixture was uniformly mixed, and then reacted at room temperature for 2 hours with intermittent mixing. N-acetylcysteine was added to the reaction system in an amount of 20 folds of the number of moles of ve-MMAE added in the reaction solution. After uniform mixing, the mixture was left to stand for 5 minutes. After a total of three instances of exchange into a conjugate storage solution (20 mM Na-citrate, 0.3% NaCl, 5% trehalose, 0.05% Tween-80, pH 6.0) by using a 15 mL 30 KD ultrafiltration device, the antibody-drug conjugate MYK-3 was obtained. The sample was stored at 4°C.

Determination of drug/antibody ratio:

The prepared antibody-drug conjugate MYK-3 was subjected to HIC-HPLC analysis (Jun Ouyang, Drug-To-Antibody (DAR) Ratio and Drug Distributionby Hydrophobic Interaction Chromatography and Reverse Phase High Performance Chromatography, Laurent Ducry (ed.), Antibody Drug Conjugates, Chapter 17, Methods in Molecular Biology, Vol 1045, p275-283) to determine the drug/antibody ratio (DAR). Referring to Figure 1, the average drug-to-antibody ratio (DAR) was 4.1, as calculated from the peak area of the chromatogram.

**Example 2:** Determination of antibody-drug conjugate MYK-3 for *in vitro* cell activity

Detection method for cell activity:

After the thawed cell line underwent 3-4 generations of cell passage, the medium was first discarded by pouring, and the remainder was rinsed once with 5 mL of DPBS and then subjected to cell digestion with 3 mL of trypsin, followed by resuspension with a medium and centrifugation with a centrifuge, respectively. The supernatant was discarded, and the remainder was then resuspended again with a medium. 0.5 mL was taken, and counting was carried out with a cell counter. After plating on a 96-well cell culture plate (DiFi cells were plated at 10000 cells/well, HT-29 cells were plated at 5000 cells/well, A549 cells were plated at 2000 cells/well, U87-MG cells were plated at 3000 cells/well, and LoVo cells were plated at 4000 cells/well) and culturing for 24 hours, a series of diluted concentrations of the monoclonal antibody BA03 and the antibody-drug conjugate MYK-3 were added, and the mixture was maintained at the temperature for 72 hours, after which 20 μL of a CCK8 color development reagent was added to each well, and OD450-650 was detected with a microplate reader at a wavelength of 450-650 nm, followed by four-parameter fitting.

Results of *in vitro* cell activity experiment:

The following cell lines were all purchased from Shanghai Institute of Cell Biology, Chinese Academy of Sciences.

Activity in DiFi cells with high EGFR expression (human colorectal cancer cells): Compared with the monoclonal

antibody BA03, MYK-3 had a significantly increased inhibitory activity on cell growth, and $EC_{50}$ was reduced by about 10 folds (the $EC_{50}$ of BA03 was 51.9 ng/mL, and the $EC_{50}$ of MYK-3 was 5.1 ng/mL), as shown in Figure 2.

Activity in other tumor cells with moderate and low EGFR expressions: Compared with the monoclonal antibody itself against cancer cells with moderate and low EGFR expressions (human colon cancer cells HT29, human lung cancer cells A549, and human astroglioblastoma cells U87-MG), MYK-3 also showed a significant inhibitory activity on cell growth (as shown in Figures 3, 4, and 5), wherein the $EC_{50}$ on HT-29 was 611 ng/mL, the $EC_{50}$ on A549 was 28.3 $\mu$g/mL, and the $EC_{50}$ on U87-MG was 5.3 $\mu$g/mL.

In addition, we also tested the activity in KRAS-mutant colon cancer cells LoVo with moderate EGFR expression (Dunn EF, Ilda M, Myers RA, Hintz KA, Campbell DA, Armstrong EA, Li C and Wheeler DL. Dasatinib sensitizes KRAS mutant colorectal tumors to cetruximab. Oncogene 2011; 30:561-574) and found that MYK-3 showed a significant tumor growth inhibitory activity on KRAS-mutant colon cancer cells LoVo (as shown in Figure 6, the $EC_{50}$ was 3.2 $\mu$g/mL), whereas BA03 had little to no inhibitory activity on this cell line when used alone.

**Example 3:** Clinical trial of antibody-drug conjugate MYK-3

The results of this trial came from the phase I clinical trial and the ongoing phase II expansion.

1) Clinical phase I

Research method:

In a dose-escalation phase of a 3 + 3 design clinical phase Ia, patients with local advanced or metastatic solid tumors who failed a standard treatment or could not get the standard treatment received MYK-3 once every 3 weeks (Q3W) for up to 8 cycles of treatment. The initial dose of MYK-3 was 0.1 mg/kg, and the subsequent doses were 0.3, 0.6, 1.0, 1.5, 2.0, 2.5, and 3.0 mg/kg. The phase Ib clinical study included extended cohorts with advanced or metastatic CRC (colorectal cancer), SCCHN (squamous cell carcinoma of the head and neck), and NPC (nasopharyngeal carcinoma). These cohorts had progressed with various previous treatments and were treated at 2.5 mg/kg Q3W. The observation results included AE (adverse event), DLT (dose-limiting toxicity), and anti-tumor activity evaluated once every two cycles.

Results:

A total of 22 patients were enrolled in clinical phase Ia without EGFR-positive pre-screening. After EGFR IHC pre-screening, a total of 39 patients suffering from CRC (12), SCCHN (13), and NPC (14) were enrolled in clinical phase Ib. The median ages of the patients in phases Ia and Ib were 54.5 and 50.4, respectively, and the median numbers of the previous treatments were 3 (1, 8) and 2 (1, 6), respectively. The dosage in Ph Ib was 2.5 mg/kg. In phase I, common TRAEs (treatment-related adverse events) occurred in ≥ 20% of patients were rash and elevated aspartate aminotransferase (39%); hair loss (33%); appetite loss (31%); elevated alanine aminotransferase (30%); pruritus (28%); decreased white blood cell count (25%); myalgia (23%); fever (21%); and decreased neutrophil count and fatigue (20%). 19 cases (31%) reported the most common TRAEs ≥ level 3. All AEs ≥ level 3 occurred in the 2.5 mg/kg cohort. In the 19 patients who received tumor evaluation in phase Ia, the ORR (objective remission rate) was 5% (1/19), and the DCR (disease control rate) was 32% (6/19). In the 27 patients who could be evaluated for efficacy in phase Ib, the BOR (best overall efficacy) was 8 cases with confirmed PR (partial remission of tumor) and 12 cases with SD (stable disease), the ORR was 29.6%, and the DCR was 74%. In the patients with SCCHN, the ORR was 40%, and the DCR was 100%; in the patients with NPC, the ORR was 44%, and the DCR was 89%; and in the patients with CRC, the ORR was 0%, and the DCR was 25%.

Conclusions:

Compared with the monoclonal antibodies targeting EGFR (including cetuximab and panitumumab) approved by FDA, MYK-3 showed a lower frequency of rash, itching and other dermatologic toxic reactions during treatment than cetuximab and panitumumab. MYK-3 showed controllable safety and encouraging anti-tumor activity in the phase I clinical study, and demonstrated an encouraging anti-tumor activity in patients with advanced EGFR-positive solid tumors that recurred or metastasized after multiple previous tumor-associated treatments (including NPC and SCCHN), which would be further studied in the phase II study. Based on the safety data of patients in the dose-escalation phase of phase Ia and the dose-expansion phase of phase Ib, the safety and tolerance of MYK-3 at a dose of 2.0 mg/kg was expected to be improved as compared with the dose of 2.5 mg/kg, and therefore, 2.0 mg/kg and 2.3 mg/kg would be further explored in the phase II study as possible clinical recommended doses of MYK-3.

2) Clinical phase II

Research method:

In the ongoing clinical phase II expansion study, a clinical study of MYK-3 in the treatment of recurrent metastatic nasopharyngeal carcinoma was carried out. The patients received MYK-3 once every 3 weeks (Q3W), and the doses used were 2.0 mg/kg and 2.3 mg/kg.

Description of trial:

A clinical trial to evaluate the potential efficacy and safety of MYK-3 in the treatment of subjects with recurrent metastatic nasopharyngeal carcinoma (NPC).

Study population:

The study included two parts: part A (an exploratory phase IIa study to confirm the effectiveness of MYK-3 in a population of NPC subjects suitable for receiving a second-line or higher treatment) and part B (a confirmatory phase IIb study in a population of NPC subjects suitable for receiving a third-line or higher treatment).

All the subjects were patients with recurrent metastatic nasopharyngeal carcinoma who were inoperable and not suitable for radiotherapy and should have the following characteristics: (1) part A: those who had received initial operation, relapsed after radiotherapy, and were not suitable for further operation or radiotherapy, and who had previously received at least a first-line platinum-containing regimen and systematic treatment with a PD-1/PD-L1 inhibitor, during which progression occurred or after which recurrence or intolerance occurred; (2) part B: those who had previously received at least a second-line systemic chemotherapy and PD-1/PD-L1 treatment and failed, with the previous chemotherapy regimen necessarily including at least a platinum-containing regimen, gemcitabine, and taxane drugs/capecitabine (the platinum-containing regimen included cisplatin, carboplatin, nedaplatin, etc.; and the taxane drugs included paclitaxel, docetaxel, albumin-bound paclitaxel, and paclitaxel liposome); (3) the disease was an incurable advanced metastatic tumor, and participating in the clinical trial was an appropriate choice of treatment; and (4) those who met all the inclusion criteria and did not met any exclusion criteria. The platinum-containing regimen included cisplatin, carboplatin, nedaplatin, etc.

Study design:

This phase II study included part A and part B.

Part A (exploratory phase): The safety and PK characteristics of MYK-3 in subjects suitable for a second-line or higher treatment would be evaluated to confirm the optimal dose and tolerance, and the ORR was evaluated by the Independent Review Committee (IRC) according to RECIST v1.1 to confirm the ineffectiveness vs anti-tumor activity of MYK-3. When the safety and anti-tumor efficacy of MYK-3 were fully proved on the basis of the recorded ORR in part A, the study entered the phase of part B.

Part B was a randomized controlled clinical study to compare the efficacy and safety of MYK-3 for injection and capecitabine/docetaxel in patients with recurrent metastatic nasopharyngeal carcinoma who had previously received at least a second-line systemic chemotherapy and PD-1/PD-L1 and failed. In this study, the principle of randomization was used, and stratified randomization was carried out according to two factors, liver metastasis (yes vs no) and ECOG PS (0 vs 1). The subjects in the experimental group would receive MYK-3 treatment. In the control group, if the subjects had not previously received capecitabine treatment, capecitabine would be preferably selected; and if they had previously received capecitabine treatment, the investigator would select capecitabine or docetaxel treatment for them.

Based on the data of the completed first-in-human trial and the subsequent analysis of efficacy, safety, PK, etc., the dose-escalation phase of phase Ia showed that the MTD was 2.5 mg/kg, and the expansion phase of phase Ib showed that this dose level achieved a better efficacy in the head and neck squamous cell carcinoma cohort and the nasopharyngeal carcinoma cohort. Considering the risks and benefits and the optimal dose evaluated suitable for further development, the previous dose level of 2.0 mg/kg of MTD and the intermediate dose of 2.3 mg/kg were selected in this phase II study as doses for further exploration in part A of this trial.

Part A: The subjects would receive 2.0 mg/kg or 2.3 mg/kg of MYK-3 intravenously on the 1st day of every 3 weeks (Q3W, 3 weeks was one treatment cycle).

Part B: The subjects in the experimental group would receive 2.3 mg/kg of MYK-3 intravenously on the 1st day of every 3 weeks (Q3W, 3 weeks was one treatment cycle). The subjects in the control group would receive 1000 mg/$m^2$ of capecitabine bid orally on the 1st-14th day of every 3 weeks (Q3W, 3 weeks was one treatment cycle) (if the time of administration for the first dose of capecitabine was the evening of D1, it was taken orally until the morning of D15) or receive 75 mg/$m^2$ of docetaxel intravenously on the 1st day of every 3 weeks (Q3W, 3 weeks was one treatment cycle), depending on the choice made by the investigator.

SMC (Safety Monitoring Committee) finally determined the recommended dose of MYK-3 in part B as 2.3 mg/kg based on the comprehensive data of long-term dosage safety, efficacy, PK, etc. in part A and the phase I trial.

Results of trial:

The results of the trial are as shown in Tables 1, 2, and 3 below.

Table 1: Efficacy of MYK-3 in subjects with recurrent metastatic nasopharyngeal carcinoma (NPC)

| Statistics | 2.0 mg/kg | 2.3 mg/kg |
|---|---|---|
| n | 28 | 29 |
| PR | 11 | 16 |
| SD | 9 | 9 |
| PD | 8 | 4 |
| NE | 2 | 2 |
| ORR (%) | 39.3 | 55.2 |
| DCR (%) | 71.4 | 86.2 |

Table 2: Statistical results of EGFR IHC in enrolled subjects with recurrent metastatic nasopharyngeal carcinoma (NPC)

| EGFR IHC | 2.0 mg/kg | 2.3 mg/kg |
|---|---|---|
| 0 (0%) | 1 | 1 |
| 1+ (1-25%) | 3 | 0 |
| 2+ (25-50%) | 2 | 4 |
| 3+ (51-75%) | 5 | 4 |
| 4+ (76-100%) | 17 | 20 |

Notes: each of the 2.0 and 2.3 mg/kg groups had two patients in which no EGFR IHC was detected.

Table 3: Summary of adverse reactions of MYK-3 in patients with recurrent metastatic nasopharyngeal carcinoma

| AE | Dose 1 (2.0 mg/kg, N = 30) (n, %) | Dose 2 (2.3 mg/kg, N = 30) (n, %) |
|---|---|---|
| All AEs | 30 (100.0) | 31 (100.0) |
| Level 1-2 | 30 (100.0) | 31 (100.0) |
| ≥ Level 3 | 13 (43.3) | 16 (51.6) |
| SAE | 5 (16.7) | 10 (32.2) |
| Treatment termination | 1 (3.3) | 2 (6.5) |
| Death | 0 | 1 (3.2) |
| Treatment-related adverse events (TRAEs) | 30 (100.0) | 29 (93.5) |
| Level 1-2 | 30 (100.0) | 29 (93.5) |
| ≥ Level 3 | 10 (33.3) | 14 (45.2) |
| SAE | 3 (10) | 6 (19.4) |
| Treatment termination | 0 | 2 (6.5) |
| Death | 0 | 0 |

Conclusions:

The exploratory phase IIa study of MYK-3 showed that in the subjects with recurrent metastatic nasopharyngeal carcinoma after multiple previous tumor-associated treatments, MYK-3 exhibited a significant anti-tumor activity, and the objective remission rates of tumor at the doses of 2.0 mg/kg and 2.3 mg/kg were 39.3% and 55.2%, respectively. This gives a new hope to such a population for whom effective treatment means lack at present. Based on the fact that the efficacy of the 2.3 mg/kg dosage group was superior to that of the 2.0 mg/kg dosage group and the safety and tolerance at the dose of 2.3 mg/kg were good, 2.3 mg/kg was finally selected as the dosage of MYK-3 for the treatment of recurrent metastatic nasopharyngeal carcinoma.

## Claims

**1.** An antibody-drug conjugate, or a pharmaceutically acceptable salt or solvate thereof, or a solvate of the salt, for use in treating an epidermal growth factor receptor (EGFR)-associated disease, wherein
the antibody-drug conjugate has a structure represented by formula I:

$$\text{Ab-(L-D)}_p \qquad \text{formula I}$$

in which:

Ab represents an anti-epidermal growth factor receptor antibody, and the anti-epidermal growth factor receptor antibody comprises a heavy chain and a light chain, wherein CDR1, CDR2, and CDR3 in a heavy chain variable region comprise the sequences as set forth in SEQ ID NO: 5, SEQ ID NO: 6, and SEQ ID NO: 7, respectively, or mutants thereof, and CDR1, CDR2, and CDR3 in a light chain variable region comprise the sequences as set forth in SEQ ID NO: 12, SEQ ID NO: 13, and SEQ ID NO: 14, respectively, or mutants thereof;
L represents a linker, and the linker is 6-maleimidocaproyl-valine-citrulline-p-aminobenzyloxycarbonyl (MC-vc-PAB);
D represents a cytotoxic agent, and the cytotoxic agent is MMAE; and
p is any numerical value selected from 1-8,
wherein the total dosage of the antibody-drug conjugate, or the pharmaceutically acceptable salt or solvate thereof, or the solvate of the salt every three weeks is 2.1-2.4 mg/kg.

**2.** The antibody-drug conjugate, or the pharmaceutically acceptable salt or solvate thereof, or the solvate of the salt according to claim 1, wherein the total dosage of the antibody-drug conjugate, or the pharmaceutically acceptable salt or solvate thereof, or the solvate of the salt every three weeks is 2.2-2.4 mg/kg; and
preferably, the total dosage of the antibody-drug conjugate, or the pharmaceutically acceptable salt or solvate thereof, or the solvate of the salt every three weeks is 2.3 mg/kg.

**3.** The antibody-drug conjugate, or the pharmaceutically acceptable salt or solvate thereof, or the solvate of the salt according to either claim 1 or 2, wherein the single dose of the antibody-drug conjugate, or the pharmaceutically acceptable salt or solvate thereof, or the solvate of the salt is 2.1-2.4 mg/kg (preferably 2.2-2.4 mg/kg, more preferably 2.3 mg/kg), and the frequency of administration is once every three weeks.

**4.** The antibody-drug conjugate, or the pharmaceutically acceptable salt or solvate thereof, or the solvate of the salt according to either claim 1 or 2, wherein the single dose of the antibody-drug conjugate, or the pharmaceutically acceptable salt or solvate thereof, or the solvate of the salt is 0.7-1.4 mg/kg, and the frequency of administration is once every 7-10 days.

**5.** The antibody-drug conjugate, or the pharmaceutically acceptable salt or solvate thereof, or the solvate of the salt according to any one of claims 1-4, wherein the mode of administration of the antibody-drug conjugate, or the pharmaceutically acceptable salt or solvate thereof, or the solvate of the salt is intravenous drip.

**6.** The antibody-drug conjugate, or the pharmaceutically acceptable salt or solvate thereof, or the solvate of the salt according to any one of claims 1-5, wherein the subject to be administered with the antibody-drug conjugate, or the pharmaceutically acceptable salt or solvate thereof, or the solvate of the salt is a human;

preferably, the subject to be administered with the antibody-drug conjugate, or the pharmaceutically acceptable

salt or solvate thereof, or the solvate of the salt is a patient suffering from an epidermal growth factor receptor (EGFR)-associated disease;
preferably, the subject to be administered with the antibody-drug conjugate, or the pharmaceutically acceptable salt or solvate thereof, or the solvate of the salt is a patient who had been previously treated with an anti-EGFR drug, and preferably, the anti-EGFR drug is an anti-EGFR antibody; and
preferably, the subject to be administered with the antibody-drug conjugate, or the pharmaceutically acceptable salt or solvate thereof, or the solvate of the salt is a patient who overexpresses EGFR.

**7.** The antibody-drug conjugate, or the pharmaceutically acceptable salt or solvate thereof, or the solvate of the salt according to claim 1 or 6, wherein the epidermal growth factor receptor (EGFR)-associated disease is a cancer;

preferably, the cancer is selected from nasopharyngeal carcinoma, head and neck squamous cell carcinoma, biliary tract cancer, esophageal cancer, duodenal cancer, colorectal cancer, colon cancer, rectal cancer, head and neck cancer, lung cancer (such as non-small cell lung cancer), ovarian cancer, cervical cancer, bladder cancer, esophagus cancer, breast cancer, renal cancer, prostate cancer, gastric cancer, pancreatic cancer, and glioma;
preferably, the cancer is selected from nasopharyngeal carcinoma, head and neck squamous cell carcinoma, non-small cell lung cancer, biliary tract cancer, gastric cancer, esophageal cancer, and duodenal cancer; and
preferably, the cancer is selected from advanced EGFR-positive and HER2-negative gastric cancer, recurrent metastatic nasopharyngeal carcinoma, recurrent or metastatic head and neck squamous cell carcinoma, advanced EGFR-positive biliary tract adenocarcinoma, and advanced EGFR-positive non-small cell lung cancer.

**8.** The antibody-drug conjugate, or the pharmaceutically acceptable salt or solvate thereof, or the solvate of the salt according to any one of claims 1-7, wherein p is any numerical value selected from 2-6; and
preferably, p is any numerical value selected from 3-5.

**9.** The antibody-drug conjugate, or the pharmaceutically acceptable salt or solvate thereof, or the solvate of the salt according to any one of claims 1-8, wherein the anti-epidermal growth factor receptor antibody has one or more of the following features:

1) FR1, FR2, FR3, and FR4 regions in a heavy chain variable region of the anti-epidermal growth factor receptor antibody comprise the sequences as set forth in SEQ ID NO: 8, SEQ ID NO: 9, SEQ ID NO: 10, and SEQ ID NO: 11, respectively, or mutants thereof;
2) FR1, FR2, FR3, and FR4 regions in a light chain variable region of the anti-epidermal growth factor receptor antibody comprise the sequences as set forth in SEQ ID NO: 15, SEQ ID NO: 16, SEQ ID NO: 17, and SEQ ID NO: 18, respectively, or mutants thereof;
3) a heavy chain constant region of the anti-epidermal growth factor receptor antibody is selected from human IgG, IgM, IgA, IgD, and IgE constant regions, or mutants of the constant regions; and preferably, the IgG is selected from IgG1, IgG2, IgG3, and IgG4; and
4) a light chain constant region of the anti-epidermal growth factor receptor antibody is human lambda constant region or kappa constant region, or a mutant of the constant region.

**10.** The antibody-drug conjugate, or the pharmaceutically acceptable salt or solvate thereof, or the solvate of the salt according to any one of claims 1-9, wherein the anti-epidermal growth factor receptor antibody has one or more of the following features:

1) the sequence of the heavy chain variable region of the anti-epidermal growth factor receptor antibody comprises the sequence as set forth in SEQ ID NO: 1, or a sequence with more than 70%, preferably more than 75%, 80%, 85%, 90%, 95%, or 99% homology with the sequence as set forth in SEQ ID NO: 1; and
preferably, the sequence of the heavy chain variable region of the anti-epidermal growth factor receptor antibody is as set forth in SEQ ID NO: 1;
2) the sequence of the light chain variable region of the anti-epidermal growth factor receptor antibody comprises the sequence as set forth in SEQ ID NO: 2, or a sequence with more than 70%, preferably more than 75%, 80%, 85%, 90%, 95%, or 99% homology with the sequence as set forth in SEQ ID NO: 2; and
preferably, the sequence of the light chain variable region of the anti-epidermal growth factor receptor antibody is as set forth in SEQ ID NO: 2;
3) the sequence of the heavy chain constant region of the anti-epidermal growth factor receptor antibody comprises the sequence as set forth in SEQ ID NO: 3, or a sequence with more than 70%, preferably more than 75%, 80%, 85%, 90%, 95%, or 99% homology with the sequence as set forth in SEQ ID NO: 3; and

preferably, the sequence of the heavy chain constant region of the anti-epidermal growth factor receptor antibody is as set forth in SEQ ID NO: 3; and
4) the sequence of the light chain constant region of the anti-epidermal growth factor receptor antibody comprises the sequence as set forth in SEQ ID NO: 4, or a sequence with more than 70%, preferably more than 75%, 80%, 85%, 90%, 95%, or 99% homology with the sequence as set forth in SEQ ID NO: 4; and
preferably, the sequence of the light chain constant region of the anti-epidermal growth factor receptor antibody is as set forth in SEQ ID NO: 4.

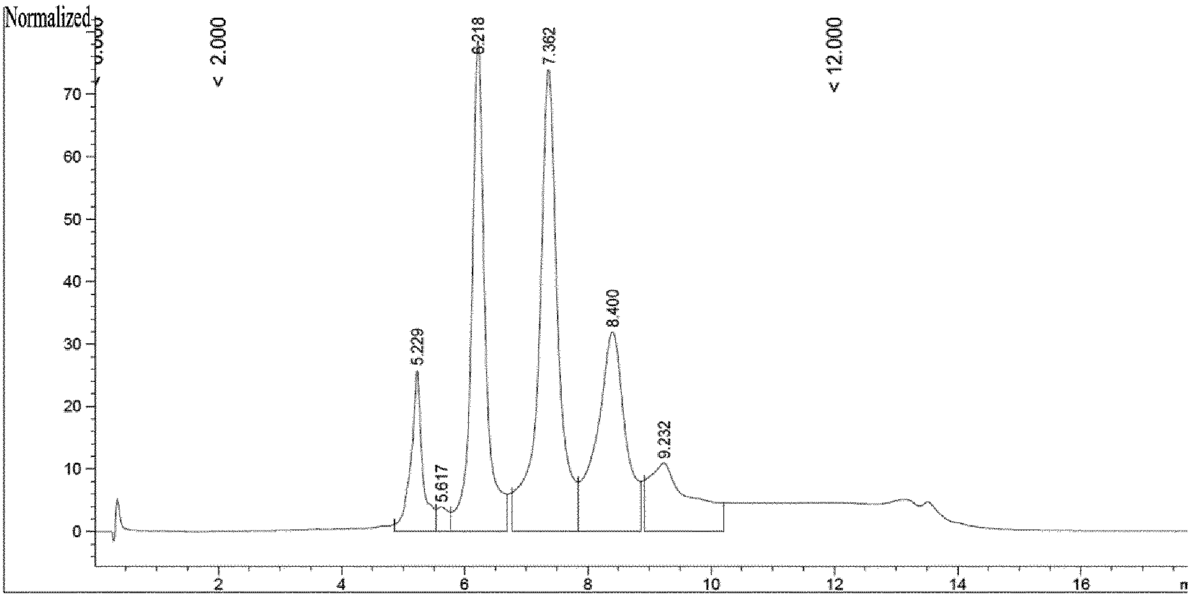


Figure 1

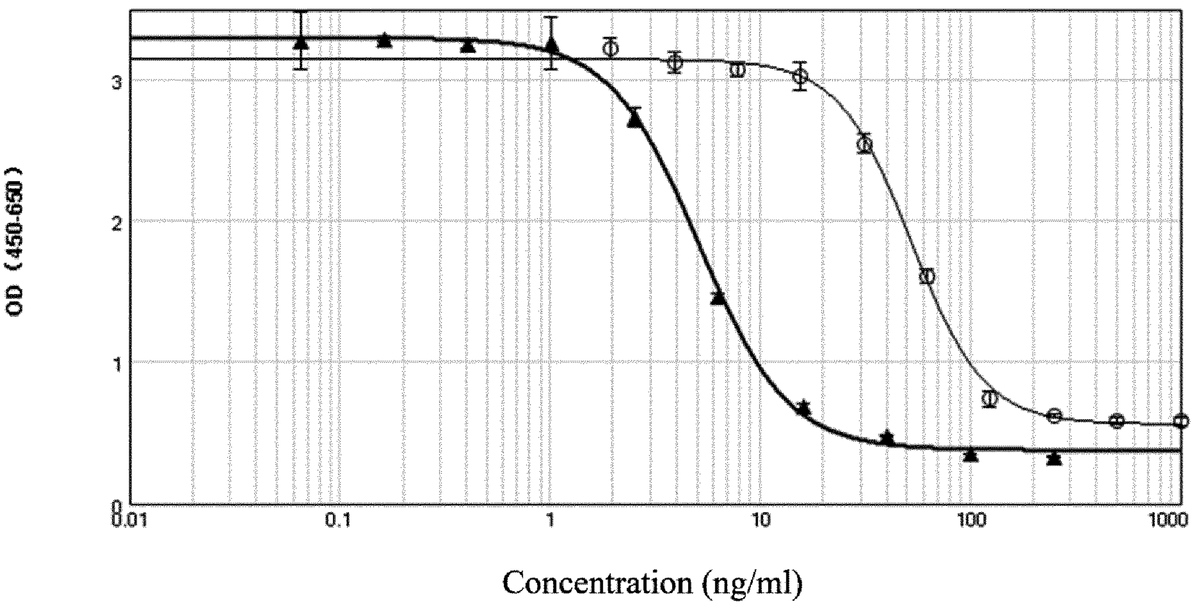


Figure 2

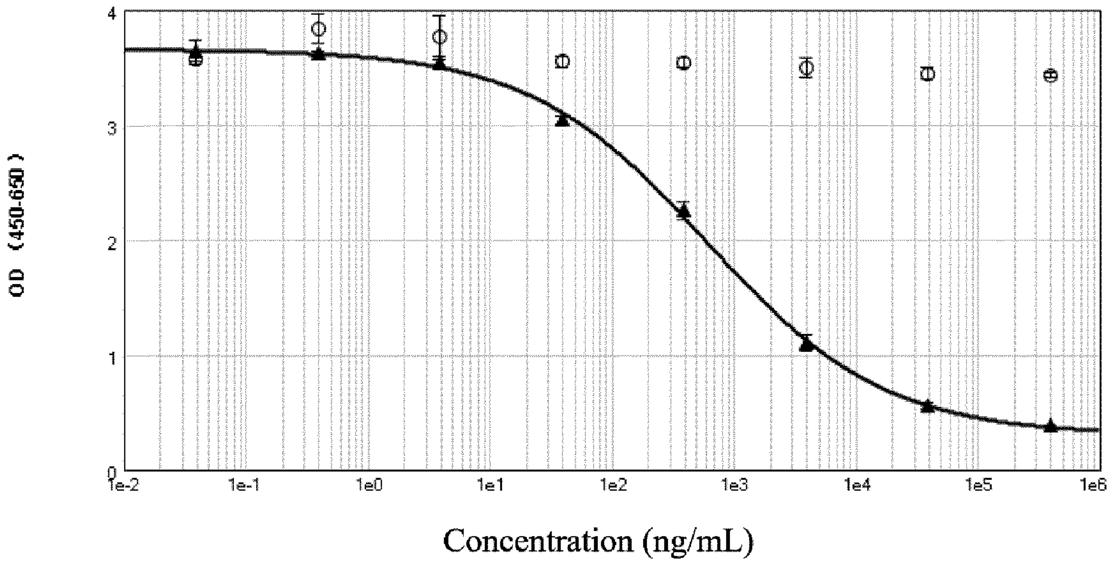


Figure 3

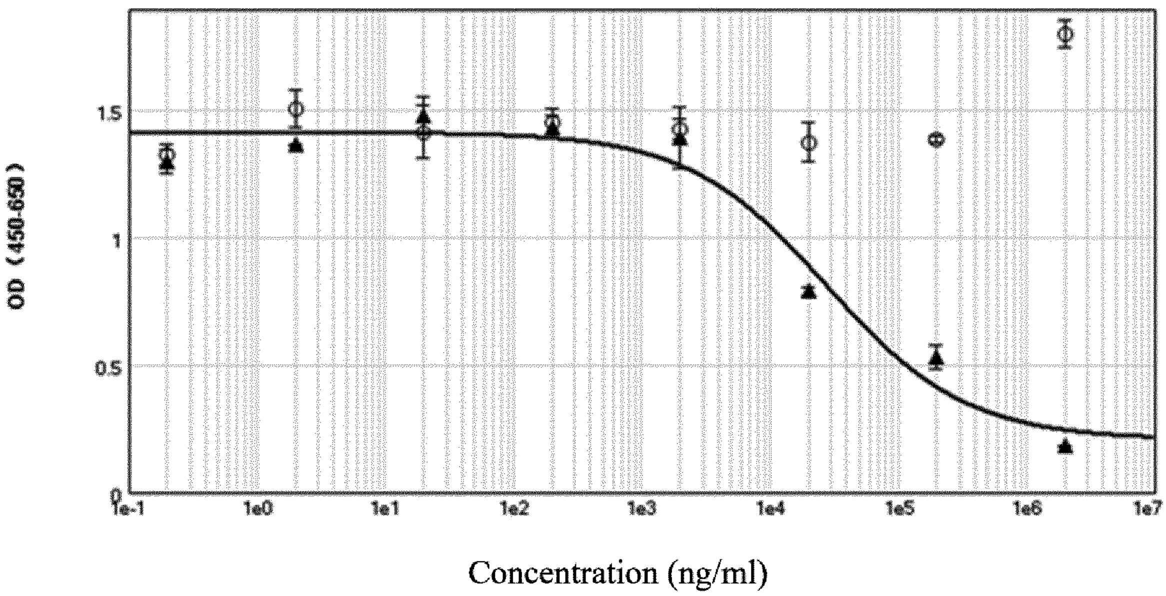


Figure 4

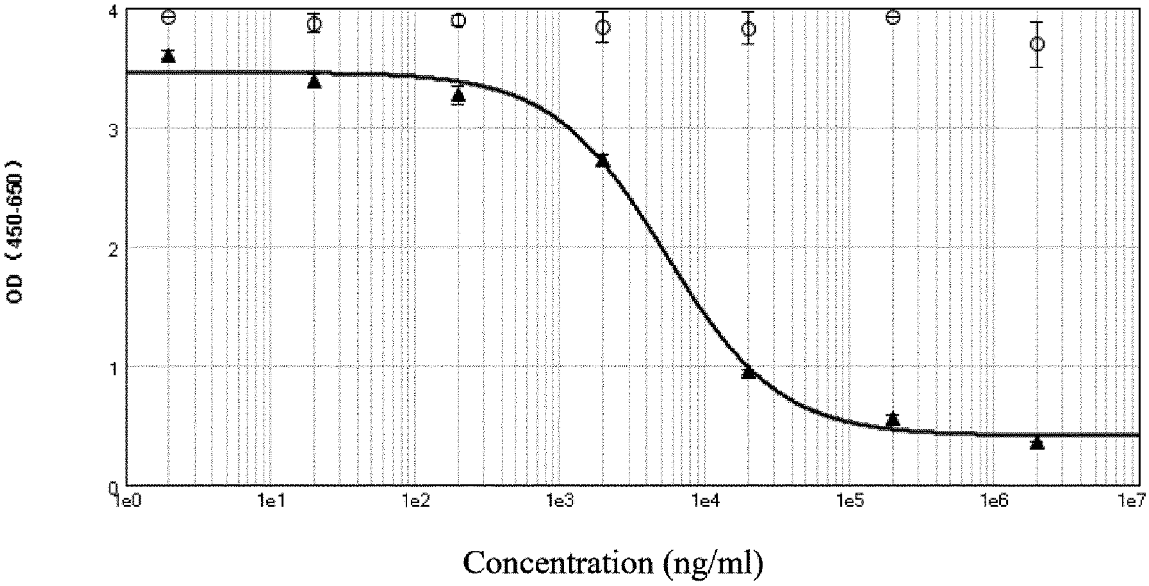


Figure 5

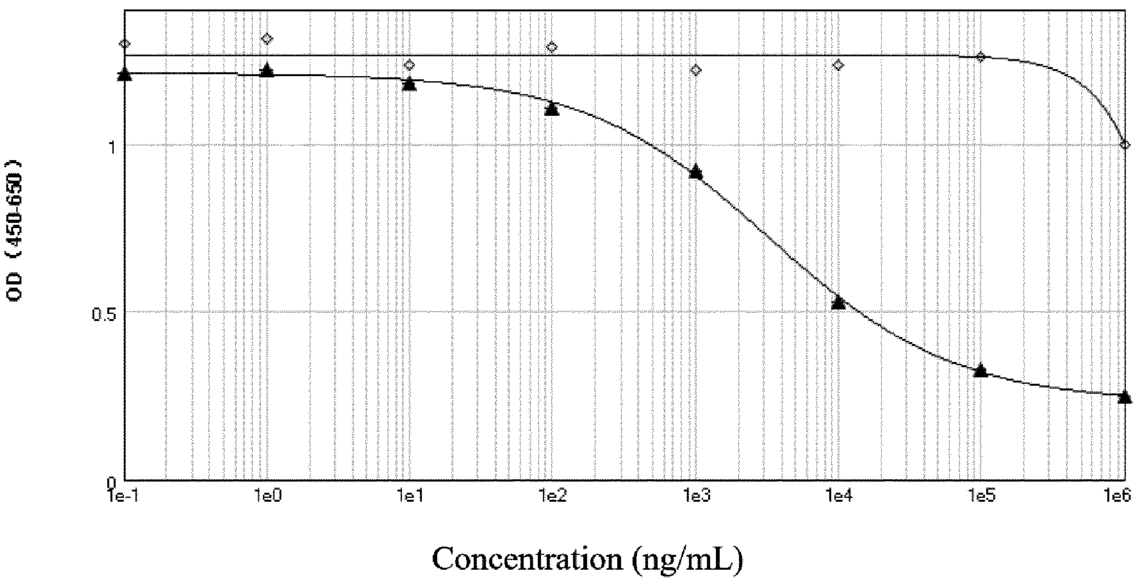


Figure 6

**INTERNATIONAL SEARCH REPORT**

International application No.
**PCT/CN2023/119471**

**A. CLASSIFICATION OF SUBJECT MATTER**

A61K47/68(2017.01)i；C07K16/30(2006.01)i；A61K39/395(2006.01)i；A61P35/00(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB OF SCIENCE, GENBANK, EMBL-EBI, STNext: 上海美雅珂生物技术有限责任公司, 乐普生物科技股份有限公司, 金永帅, 胡朝红, 隋滋野, 抗体药物偶联物, antibody-drug conjugat, 表皮生长因子受体, epidermal growth factor receptor, EGFR, 细胞毒剂, MMAE, CDR, 溶剂化物, solvate, 癌症, cancer, 肿瘤, tumor, SEQ ID NOs: 1-18

**C. DOCUMENTS CONSIDERED TO BE RELEVANT**

| Category* | Citation of document, with indication, where appropriate, of the relevant passages | Relevant to claim No. |
|---|---|---|
| X | CN 111529717 A (SHANGHAI MIRACOGEN INC.) 14 August 2020 (2020-08-14) description, paragraphs 23-50, 58-59, and 89-91 | 1-10 |
| PX | CN 115814104 A (SHANGHAI MIRACOGEN INC.) 21 March 2023 (2023-03-21) claims 1-10 | 1-10 |
| PX | CN 115814105 A (SHANGHAI MIRACOGEN INC.) 21 March 2023 (2023-03-21) claims 1-10 | 1-10 |
| PX | WO 2023039778 A1 (SHANGHAI MIRACOGEN INC.) 23 March 2023 (2023-03-23) claims 1-10 | 1-10 |
| A | WO 2022078523 A1 (SHANGHAI MIRACOGEN INC. et al.) 21 April 2022 (2022-04-21) claims 1-15 | 1-10 |
| A | WO 2021190480 A1 (SHANGHAI HANSOH BIOMEDICAL CO., LTD. et al.) 30 September 2021 (2021-09-30) claims 1-24 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 December 2023** | **13 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/119471**

**C. DOCUMENTS CONSIDERED TO BE RELEVANT**

| Category* | Citation of document, with indication, where appropriate, of the relevant passages | Relevant to claim No. |
|---|---|---|
| A | JÄGER, S. et al. "EGFR binding Fc domain-drug conjugates: stable and highly potent cytotoxic molecules mediate selective cell killing" *BIOL. CHEM.*, Vol. 403, 20 September 2021 (2021-09-20), page 525, and abstract | 1-10 |
| A | HU,X. Y. et al. "An EGFR-targeting antibody–drug conjugate LR004-VC-MMAE: potential in esophageal squamous cell carcinoma and other malignancies" *MOLECULAR ONCOLOGY,* Vol. 13, 15 November 2018 (2018-11-15), page 251, section 3.1 | 1-10 |

Form PCT/ISA/210 (second sheet) (July 2022)

**INTERNATIONAL SEARCH REPORT**

International application No.
**PCT/CN2023/119471**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/119471**

| Patent document cited in search report | | | Publication date (day/month/year) | Patent family member(s) | | | Publication date (day/month/year) |
|---|---|---|---|---|---|---|---|
| CN | 111529717 | A | 14 August 2020 | WO | 2016131409 | A1 | 25 August 2016 |
| | | | | US | 2018036423 | A1 | 08 February 2018 |
| | | | | US | 10792370 | B2 | 06 October 2020 |
| CN | 115814104 | A | 21 March 2023 | | None | | |
| CN | 115814105 | A | 21 March 2023 | | None | | |
| WO | 2023039778 | A1 | 23 March 2023 | | None | | |
| WO | 2022078523 | A1 | 21 April 2022 | CO | 2023005979 | A2 | 29 May 2023 |
| | | | | EP | 4230225 | A1 | 23 August 2023 |
| | | | | IL | 302122 | A | 01 June 2023 |
| | | | | AU | 2021359562 | A1 | 25 May 2023 |
| | | | | CA | 3198667 | A1 | 21 April 2022 |
| WO | 2021190480 | A1 | 30 September 2021 | TW | 202144012 | A | 01 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 106999606 B
- CN 103772504 A


### Non-patent literature cited in the description


- Kabat Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- **ALTSCHUL et al.** *Nucl. Acid. Res.*, 1977, vol. 25, 3389-3402
- **ALTSCHUL et al.** *J. Mol. Biol.*, 1990, vol. 215, 403-410
- **WILD, R. et al.** *Mol. Cancer Rher*, 2006, vol. 5 (1), 104-113
- Immunology-A Synthesis. Sinauer Associates, 1991
- Drug-To-Antibody (DAR) Ratio and Drug Distributionby Hydrophobic Interaction Chromatography and Reverse Phase High Performance Chromatography. **JUN OUYANG**. Methods in Molecular Biology, vol. 1045, 275-283
- **DUNN EF** ; **ILDA M** ; **MYERS RA** ; **HINTZ KA** ; **CAMPBELL DA** ; **ARMSTRONG EA** ; **LI C** ; **WHEELER DL**. Dasatinib sensitizes KRAS mutant colorectal tumors to cetruximab.. *Oncogene*, 2011, 561-574